# EUROPÄISCHE PATENTSCHRIFT

(11) **EP 1 277 432 B1**
(45) Veröffentlichungstag und Bekanntmachung des Hinweises auf die Patenterteilung: **11.07.2007**
(21) Anmeldenummer: 01117268.1
(22) Anmeldetag: 17.07.2001
(51) Int. Cl.: A61B 1/24, A61B 1/04

(54) **Anordnung zum Betrachten und Aufnehmen von Objekten im Mund eines Patienten und Betriebsverfahren**
Apparatus for viewing and taking pictures of objects in the mouth of a patient and processing
Appareil permettant de visualiser et de prendre de vues d'objets dans la bouche d'un patient et procédé

(43) Veröffentlichungstag der Anmeldung: 22.01.2003
(73) Patentinhaber: Orangedental GmbH & Co. KG, 88400 Biberach (DE)
(72) Erfinder: Piernitzki, Bernhard, 19089 Demen (DE)
(74) Vertreter: Habersack, Hans-Jürgen

(56) Entgegenhaltungen:
- DE-U- 29 621 838
- US-A- 5 661 519
- US-B1- 6 224 542

## Beschreibung

Die Erfindung betrifft eine Anordnung zum Aufnehmen einzelner Bilder von Objekten im Mund eines Patienten mit einer Intraoralkamera zum kontinuierlichen Aufnehmen von Bildern aus dem Mund des Patienten, einem mit der Intraoralkamera verbundenes Anzeigegerät zur Darstellung der aufgenommenen Bilder und einer Speichereinrichtung zum Abspeichern eines Einzelbildes aus den auf dem Anzeigegerät kontinuierlich dargestellten Bildern auf einen Auslösebefehl hin. Sie betrifft weiter ein Verfahren zum Betrieb einer derartigen Anordnung.

Im zahnärztlichen Bereich werden oft anstelle von kleinen Winkelspiegeln sogenannte Intraoralkameras zum Betrachten und Aufnehmen von Bilder aus dem Mund eines Patienten verwendet. Diese bestehen in der Regel aus einem Griffstück mit einer schmalen zylindrischen Verlängerung, die die Aufnahmeoptik, beispielsweise eine Faseroptik trägt.

Eine solche Intraoralkamera bietet eine einfache und bequeme Möglichkeit, alle Bereiche im Inneren des Mundes abzubilden. Sie kann wegen ihrer kleiner Bauweise auch solche Gebiete abbilden, die ansonsten nur schwer zugänglich sind, wie etwa Wurzelkanäle oder Bereiche mit tiefer Karies.

Sie ermöglicht auch die Beobachtung des Fortgangs eines zahnärztlichen Eingriffs an einem Monitor in Echtzeit durch den behandelnden Arzt selbst, aber beispielsweise auch durch eine Gruppe von Studenten zur Aus- oder Weiterbildung.

Darüber hinaus können die aufgenommen Bilder mit einem Rechner auf einen beliebigen Speichermedium, etwa einer Diskette, einer Compact Disk (CD) oder einer Digital Versatile Disk (DVD) dauerhaft gespeichert werden und stehen dann zur Dokumentation einer Behandlung, beispielsweise für die Patientienkartei oder zur Antragstellung bei einer Krankenkasse jederzeit zum Ausdruck zur Verfügung.

Ein allgemeine Schwierigkeit, die bei derartigen Intraoralkameras auftritt, ergibt sich aus dem begrenzten Raum, der im Inneren des Mundes eines Patienten zur Verfügung steht, sowie der Tatsache, daß Betrachten und Aufnehmen von Bildern für den Patienten mit möglichst geringen Beeinträchtigungen verbunden sein sollen. Durch diese Einschränkungen ist es oft nicht möglich, die Kamera in eine Position zu bringen, die vom Gesichtspunkt der Bildaufnahme her optimal wäre.

Eine Kamera zur Fertigung von Aufnahmen im zahnärztlichen Bereich ist aus der DE 296 21 838 U1 bekannt. Die Betätigungseinrichtung für Aufnahmen ist dort als externer Fußschalter bzw. drahtlos über eine Funk- oder Infrarotfernsteuerung vorgesehen, was einen erhöhten Hardwareaufwand mit sich bringt und im Falle einer Fernbedienung auch die Betriebssicherheit beeinträchtigen kann.

Die US 5,661,519 A zeigt eine Videokamera, die über eine automatische Richtigstellung der Orientierung der aufgenommenen Bilder verfügt, wofür ein Positionssensor vorgesehen ist.

Nachteilig ist bei den bekannten Intraoralkameras, dass bei manueller Betätigung von Positionsschaltern, Tasten und dergleichen stets die Gefahr besteht, das aufgenommene Bild durch den Bedienvorgang selbst zu verwackeln. Dies ist besonders dann von Nachteil, wenn durch die Schalter- oder Tastenbetätigung eine Speicherung des aktuell angezeigten Bildes bewirkt werden soll. Das gespeicherte Bild kann dann durch die Bewegung unscharf geworden sein, oder einen anderen als den eigentlich gewünschten Bildausschnitt darstellen.

Hier setzt die Erfindung an. Der Erfindung, wie sie in den Ansprüchen gekennzeichnet ist, liegt die Aufgabe zugrunde, die Nachteile des genannten Stands des Technik zu vermeiden; insbesondere soll eine Intraoralkamera geschaffen werden, bei der angezeigte Bilder ohne Verwackeln abgespeichert werden können.

Diese Aufgabe wird erfindungsgemäß durch die Anordnung zum Aufnehmen einzelner Bilder von Objekten im Mund eines Patienten nach Anspruch 1, und das Verfahren zum Aufnehmen bzw. Verwenden einzelner Bilder nach Anspruch 8 gelöst. Weitere Ausgestaltungen der Erfindung gehen aus den Unteransprüchen hervor.

Erfindungsgemäß ist bei einer Anordnung zum Aufnehmen einzelner Bilder von Objekten im Mund eines Patienten mit den Merkmalen des Oberbegriffs von Anspruch 1, die Intraoralkamera mit einem - zum Beispiel in einer Recheneinheit angeordneten - Zwischenspeicher zum Zwischenspeichern zumindest eines der kontinuierlich, d.h. ständig aufgenommenen Bilder verbunden. Vorzugsweise werden die ständig aufgenommenen Bilder auf einer Darstellungseinrichtung, z.b. einem Monitor angezeigt. Auf einen Auslösebefehl hin wird ein mit einer gegenüber dem Auslösebefehl vorbestimmten Zeitvoreilung aufgenommenes Bild aus dem Zwischenspeicher entnommen und zur Weiterbehandlung bzw. Auswertung geleitet, z.B. an die Speichereinrichtung und/oder den Monitor. Der Zwischenspeicher kann auch in der Kamera selbst angeordnet sein. Der Auslösebefehl wird vorzugsweise mittels einer, insbesonbdere an der Kamera oder Kamerahalterung angebrachten Auslösetaste generiert.

Dieser Aspekt der Erfindung beruht also auf dem Gedanken, auf einen Auslösebefehl hin nicht das aktuelle Bild abzuspeichern, das durch einen mechanischen Auslösevorgang verwackelt oder verschoben sein könnte, sondern ein zuvor aufgenommenes und in dem Zwischenspeicher abgelegtes Bild, das gegenüber dem Auslösezeitpunkt eine bestimmte Zeitvoreilung aufweist.

Diese Zeitvoreilung wird mit Vorteil so gewählt, daß einerseits das aufgenommene Bild vom Auslösevorgang nicht mehr betroffen ist, daß andererseits noch ein direkter zeitlicher Bezug zum gegenwärtig angezeigten Bild besteht.

In der Praxis werden vorzugsweise die kontinuierlich aufgenommenen Bilder über eine Zeitspanne von 0,05 bis 2 Sekunden, bevorzugt von 0,1 bis 0,5 Sekunden zwischengespeichert. Besonders gut geeignet ist eine Zeitspanne von etwa 0,2 bis etwa 0,35 Sekunden. Das ausgewählte zur Weiterverwendung bestimmte Bild entspricht dann für alle praktischen Belange dem zuletzt angezeigten Bild, ist aber von möglichen Störungen durch den Auslösebefehl nicht betroffen.

Der Zwischenspeicher ist vorzugsweise als Schieberegister ausgebildet, das bei Speicherung des zuletzt aufgenommenen Bildes das älteste im Register befindliche Bild herausschiebt.

In einer zweckmäßigen Ausgestaltung enthält die Intraoralkamera eine Auslösevorrichtung, insbesondere eine Taste, deren Betätigung den Auslösebefehl bewirkt.

In einer bevorzugten Weiterbildung der Erfindung enthält die Anordnung weiter eine Einrichtung zur Lage- und Orientierungsbestimmung der Intraoralkamera und Mittel zur Übermittlung der ermittelten Lage- und Orientierungssignale an das Anzeigegerät und/oder die Recheneinheit zur seitenrichtigen und/oder aufrechten Darstellung der aufgenommenen Bilder.

Die Lagebestimmung erfolgt zweckmäßig durch Feststellung der Gravitationsrichtung, insbesondere mittels eines Quecksilberschalters, oder durch Licht.

Bei einer derartige Anordnung kann jedoch auch, wie beim ersten Aspekt der Erfindung, ein Mikrofon vorgesehen sein, das gesprochene Steueranweisungen zur Bildlage und Bildorientierung aufnimmt und an eine mit dem Anzeigegerät verbundene Recheneinheit leitet, die entsprechend den Steueranweisungen die aufgenommenen Kamerabilder gedreht und/oder gespiegelt auf dem Anzeigegerät darstellt.

Weitere vorteilhafte Ausgestaltungen, Merkmale und Details der Erfindung ergeben sich aus den abhängigen Ansprüchen, der Beschreibung der Ausführungsbeispiele und den Zeichnungen.

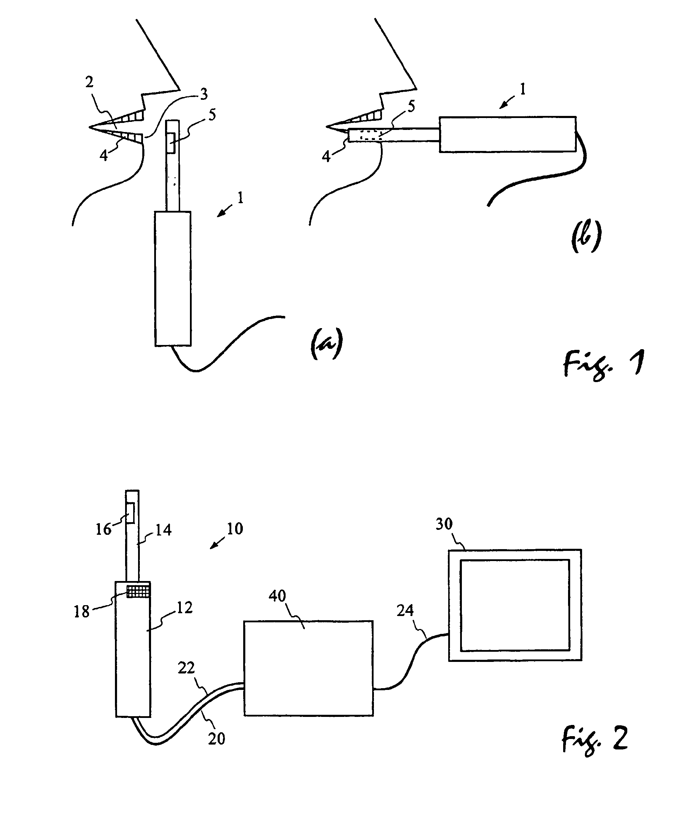

Die Erfindung soll nachfolgend anhand eines Ausführungsbeispiels im Zusammenhang mit den Zeichnungen näher erläutert werden. Es sind jeweils nur die für das Verständnis der Erfindung wesentlichen Elemente dargestellt. Dabei zeigt
- Figur 1: eine schematische Darstellung einer Ausführungsform der Erfindung.

Die in Fig. 1 gezeigte Anordnung umfaßt ebenfalls eine Intraoralkamera 10 mit einem Griffstück 12 und einer Verlängerung 14, an deren Ende sich eine Aufnahmeoptik 16 befindet. Die aufgenommen Bilder werden über eine Verbindungsleitung 20 an eine Recheneinheit 50, und von dieser über eine Verbindungsleitung 24 auf einen Monitor 30 zur Darstellung geleitet. Die Besonderheit der Kamera liegt darin, dass sie ständig Bilder, vorzugsweise mit einer bestimmten Aufnahmerate von z.B. 2 bis 25 Bildern/Sekunde aufnimmt.

Die Kamera 10 weist eine Drucktaste 26 auf, deren Betätigung zur Auswahl, z.B. Speicherung eines Bildes führt. Um zu vermeiden, daß die Betätigung der Taste 26 selbst zu einem Verwackeln oder Verschieben des Bildes führt, enthält die Recheneinheit 50 einen Zwischenspeicher 52 in dem ständig wenigstens ein Bild zwischengespeichert wird, womit sich immmer wenigstens das zuletzt aufgenommene Bild, vorzugsweise 2 bis 10 zuletzt aufgenommene Bilder, im Zwischenspeicher befindet bzw. befinden.

Im Ausführungsbeispiel ist der Zwischenspeicher 52 als FIFO-(First-In-First-Out) Stack mit einer Kapazität von 8 Videobildern organisiert. Nach Initialierung werden die von der Kamera 10 mit einer Rate von 25 Bildern pro Sekunde gelierten Bilder sukzessive in den 8 Einheiten breiten Stack geschoben.

Ist der Stack 52 mit acht Bildern gefüllt, wird jeweils beim Einschieben eines neuen Bildes das älteste Bild herausgeschoben. Somit enthält der Stack jeweils acht Bilder, die gegenüber dem momentanen Zeitpunkt um 1/25, 2/25, ... 8/25 Sekunden zeitversetzt sind.

Wird nun die Taste 26 an der Intraoralkamera 10 durch den Bediener gedrückt, läuft ein entsprechender Auslösebefehl über die Signalleitung 28 zur Recheneinheit 50. Dort wird ein innerhalb einer definerten Zeitspanne vorher aufgenommenes Bild, z.b. das älteste im Stack 52 befindliche Videobild an die Speichereinheit 60, die im Ausführungsbeispiel durch eine Festplatte gebildet ist, geleitet und abgespeichert.

Somit wird nicht das gerade am Monitor dargestellte Bild gespeichert, sondern ein Bild, dessen Aufnahmezeitpunkt 8/25 = 0,32 Sekunden zurückliegt. Verwacklungen oder Verschiebungen des abzuspeichernden Bildausschnitts durch die Tastenbetätigung sind damit ausgeschlossen.

In einer sehr preisgünstigen Ausführungsform kann auch nur ein Stack bzw. zwei Stacks bei einer Aufnahmefrequenz von 4 Bildern/sekunde vorgesehen sein, in welchem Fall das im Zwischenspeicher befindliche Bild um 0,25 oder 0,5 Sekunden voreilt.

## Patentansprüche

1. Anordnung zum Aufnehmen einzelner Bilder von Objekten im Mund eines Patienten mit einer Intraoralkamera (10) zum Aufnehmen von Bildern aus dem Mund des Patienten,
**dadurch gekennzeichnet, daß**
die Intraoralkamera ausgebildet ist zum kontinuierlichen Aufnehmen von Bilder, insbesondere mit einer definierten Aufnahmerate,
die Intraoralkamera (10) mit einem als Schieberegister ausgebildeten Zwischenspeicher (52) zum Zwischenspeichern der kontinuierlich aufgenommenen Bilder verbunden ist, welcher bei Speicherung des zuletzt aufgenommenen Bildes das älteste im Register befindliche Bild herausschiebt, und
eine Auslöseeinrichtung (26) vorgesehen ist, auf deren Signal hin eine in der Kamera oder in einer Recheneinheit (50) vorgesehene Steuerung ein Bild aus dem Zwischenspeicher entnimmt und zu einer Auswertung, z.b. Darstellung und Speicherung (30,50) leitet, welches Bild eine vorbestimmte Zeitspanne vor der Abgabe des Signals der Auslöseeinrichtung aufgenommen worden ist.

2. Anordnung nach Anspruch 3, umfassend ein mit der Intraoralkamera (10) verbundenes Anzeigegerät (30) zur Darstellung der aufgenommenen Bilder.

3. Anordnung nach Anspruch 1 oder 2, bei der die Intraoralkamera (10) die Auslöseeinrichtung eine Taste (26), aufweist, deren Betätigung das Auslösesignal generiert.

4. Anordnung nach Anspruch 1, 2 oder 3, bei der der Zwischenspeicher (52) als Schieberegister ausgebildet und derart eingerichtet und betreibbar ist, daß er die kontinuierlich aufgenommenen Kamerabilder über eine Zeitspanne von bis zu 2 Sekunden, bevorzugt von bis zu 0,5 Sekunden, besonders bevorzugt von bis zu etwa 0,2 bis zu etwa 0,35 Sekunden zwischenspeichert.

5. Anordnung nach einem der vorhergehenden Ansprüche, mit einer Einrichtung zur Lage- und Orientierungsbestimmung der Intraoralkamera (10) und mit Mitteln zur Übermittlung der bestimmten Lage- und Orientierungssignale an das Anzeigegerät (30) und/oder die Recheneinheit (50) zur seitenrichtigen Darstellung der aufgenommenen Bilder.

6. Anordnung nach Anspruch 5, bei der die Lage- und Orientierungsbestimmung durch Feststellung der Gravitationsrichtung, insbesondere mittels eines Quecksilberschalters, oder durch Licht erfolgt.

7. Anordnung nach einem der Ansprüche 1 bis 4, bei der ein Mikrofon (18) vorgesehen ist, das gesprochene Steueranweisungen zur Bildlage und Bildorientierung aufnimmt und an eine mit dem Anzeigegerät (30) verbundene Recheneinheit (50) leitet, die entsprechend den Steueranweisungen die aufgenommenen Kamerabilder gedreht und/oder gespiegelt auf dem Anzeigegerät (30) darstellt.

8. Verfahren zum Aufnehmen einzelner Bilder von Objekten im Mund eines Patienten, mit den Verfahrensschritten:
- kontinuierliches Aufnehmen von Bildern aus dem Mund des Patienten,
- Zwischenspeichern der aufgenommenen Bilder in einem als Schieberegister ausgebildeten Zwischenspeicher, der bei Speicherung des zuletzt aufgenommenen Bildes das älteste im Register befindliche Bild herausschiebt, und
- Auswerten bzw. Weiterverwenden eines eine vorbestimmte Zeitspanne vor einem Auslösebefehl aufgenommenen Einzelbildes aus dem Zwischenspeicher.

9. Verfahren nach Anspruch 8, bei dem die aufgenommenen Bilder auf einem Anzeigegerät dargestellt werden.

10. Verfahren nach Anspruch 8 oder 9, bei dem der Auslösebefehl durch einen Tastendruck an einer zur kontinuierlichen Aufnahme der Bilder vorgesehenen Intraoralkamera bewirkt wird.

11. Verfahren nach Anspruch 8, 9 oder 10, bei der die kontinuierlich aufgenommen Bilder über einer Zeitspanne von 0,05 bis 2 Sekunden, bevorzugt von 0,1 bis 0,5 Sekunden, besonders bevorzugt von etwa 0,2 bis etwa 0,35 Sekunden zwischengespeichert werden.

12. Verfahren nach einem der Ansprüche 8 bis 11, bei dem die Lage und Orientierung einer zur Aufnahme der Bilder vorgesehenen Intraoralkamera im Raum bestimmt wird, die Lage- und Orientierungssignale an das Anzeigegerät und/oder eine Recheneinheit übermittelt werden, und
die aufgenommen Bilder auf Grundlage dieser Signale seitenrichtig und/oder aufrecht dargestellt werden.

13. Verfahren nach Anspruch 12, bei dem die Lage und Orientierung der Kamera durch Feststellung der Gravitationsrichtung oder durch Licht bestimmt wird.

14. Verfahren nach einem der Ansprüche 8 bis 11, bei dem
gesprochene Steueranweisungen zur Bildlage und Bildorientierung aufgenommen werden, an eine mit dem Anzeigegerät verbundene Recheneinheit geleitet werden, und die aufgenommenen Kamerabilder entsprechend den Steueranweisungen gedreht und/oder gespiegelt darstellt werden.

## Claims

1. An assembly for capturing discrete images of objects in the mouth of a patient comprising an intraoral camera (10) for continual intraoral imaging, **characterized in that**
the intraoral camera is configured for a continual image capture, particularly at a predetermined capture rate,
the intraoral camera (10) is connected to a cache (52) configured as shift register for caching the continually captured images which in saving the image last captured shifts the stalest image in the register out thereof, and
a release button (26) is provided, in response to the signal of which a controller provided in the camera or in an arithmetic unit (50) retrieves an image from the cache and directs it to a use, e.g. display and memory (30, 50), said image being captured in a predetermined time window before output of the signal to the release means.

2. The assembly as set forth in claim 1, comprising connected to the intraoral camera (10) a monitor (30) for displaying the captured images.

3. The assembly as set forth in claim 1 or 2 wherein the intraoral camera (10) comprises a release button (26) as the release means, actuation of which generates the release signal.

4. The assembly as set forth in claim 1, 2 or 3 wherein the cache (52) is configured as a shift register and structured for operation such that it caches the continual captured camera images in a time window of up to 2 seconds, preferably up to 0.5 of a second, particularly preffered up to approx 0.2 to approx. 0.35 of a second.

5. The assembly as set forth in any of the preceding claims comprising a means for mapping the position and orientation of the intraoral camera (10) and means for communicating the mapped position and orientation signals to the display (30) and/or the arithmetic unit (50) for displaying the captured images right way round and/or up.

6. The assembly as set forth in claim 5 wherein mapping the position and orientation is done by sensing the direction of gravity, particularly by means of a mercury switch, or by light.

7. The assembly as set forth in any of the claims 1 to 4 wherein a microphone (18) is provided which records spoken control instructions as to the position and orientation of the image and communicates them to an arithmetic unit (50) connected to the display (30), the arithmetic unit then turning and/or mirroring the captured camera images for display on the monitor (30) om accordanc with the control instructions.

8. A method of capturing discrete images of objects in the mouth of a patient comprising the steps:
- continually capturing intraoral images,
- caching the captured images in a cache configured as a shift register which on saving the image last captured shifts out the oldest image in the register, and
- evaluating or making further use of a discrete image captured in a predetermined time window before a release instruction from the cache.

9. The method as set forth in claim 8 wherein the captured images are displayed on a monitor.

10. The method as set forth in claim 8 or 9 wherein the release instruction is initiated by pressing a button of an intraoral camera provided for continual imaging.

11. The method as set forth in claim 8, 9 or 10 wherein the continually captured images are preferably cached in a time window of 0.05 to 2 seconds, preferably 0.1 to 0.5 of a second, particularly suitable being a time window of approx. 0.2 to approx. 0.35 of a second.

12. The method as set forth in any of the claims 8 to 11 wherein the position and orientation of an intraoral camera provided for imaging is mapped three-dimensionally, the position and orientation signals are communicated to the display and/or arithmetic unit and the captured images are displayed on the basis of these signals right way round and/or up.

13. The method as set forth in claim 12 wherein the position and orientation of the camera is mapped by sensing the direction of gravity, or by light.

14. The method as set forth in any of the claims 8 to 11 wherein spoken control instructions as to the position and orientation of the image are recorded and communicated to an arithmetic unit connected to the display, the captured camera images being displayed turned and/or mirrored in accordance with the control instructions.

## Revendications

1. Dispositif destiné à prendre des vues individuelles d'objets situés dans la cavité buccale d'un patient à l'aide d'une caméra intraorale (10) destinée à prendre des vues provenant de la cavité buccale du patient,
**caractérisé par le fait que**
la caméra intraorale est conçue pour la prise de vues en continu, notamment à une fréquence de prise définie,
la caméra intraorale (10) est reliée à une mémoire intermédiaire (52) conçue sous la forme d'un registre à décalage destiné à l'enregistrement temporaire des vues prises en continu, ladite mémoire expulsant du registre, lors de l'enregistrement de la vue prise en dernier, la vue s'y trouvant depuis le plus longtemps, et
un dispositif de déclenchement (26) est prévu, au signal duquel une commande prévue dans la caméra ou dans une unité de calcul (50) extrait une vue de la mémoire intermédiaire et l'achemine vers une évaluation, par ex. l'affichage et l'enregistrement (30, 50), ladite vue ayant été prise à une période de temps prédéfinie avant l'émission du signal du dispositif de déclenchement.

2. Dispositif selon la revendication 3,
comprenant un dispositif de visualisation (30) relié à la caméra intraorale (10) destiné à afficher les vues prises.

3. Procédé selon la revendication 1 ou 2,
dans lequel la caméra intraorale (10) comporte un dispositif de déclenchement (26) sous forme de touche dont l'actionnement génère le signal de déclenchement.

4. Procédé selon la revendication 1, 2 ou 3,
dans lequel la mémoire intermédiaire (52) est conçue sous la forme d'un registre à décalage et est installée et exploitable de telle sorte qu'elle enregistre temporairement les vues prises par la caméra en continu sur une période de temps allant jusqu'à 2 secondes, préférentiellement jusqu'à 0,5 seconde, très préférentiellement jusqu'à environ 0,2 à environ 0,35 seconde.

5. Dispositif selon l'une des revendications précédentes,
comprenant un équipement destiné à déterminer la position et l'orientation de la caméra intraorale (10) et des moyens destinés à transmettre les signaux de position et d'orientation définis au dispositif de visualisation (30) et/ou à l'unité de calcul (50) dans de but d'afficher les vues prises à l'endroit.

6. Dispositif selon la revendication 5,
dans lequel la définition de la position et de l'orientation s'effectue par la détection du sens gravitationnel, notamment à l'aide d'un interrupteur à mercure, ou par la lumière.

7. Dispositif selon une des revendications 1 à 4,
dans lequel est prévu un microphone (18) enregistrant les instructions de commande parlées destinées à la position et à l'orientation de la vue et amenant à une unité de calcul (50) reliée au dispositif de visualisation (30), ladite unité de calcul affichant les vues prises par la caméra sur le dispositif de visualisation, en fonction des instructions de commande, après rotation et/ou de manière inversée (30).

8. Procédé destiné à prendre des vues individuelles d'objets situés dans la cavité buccale d'un patient, comportant les étapes suivantes :
- prise de vues en continu provenant de la cavité buccale du patient,
- enregistrement temporaire des vues prises dans une mémoire intermédiaire conçue sous la forme d'un registre à décalage, ladite mémoire expulsant du registre, lors de l'enregistrement de la vue prise en dernier, la vue s'y trouvant depuis le plus longtemps, et
- évaluation ou exploitation d'une vue individuelle située dans la mémoire intermédiaire prise à une période de temps prédéfinie avant un ordre de déclenchement.

9. Procédé selon la revendication 8,
dans lequel les vues prises sont affichées sur un dispositif de visualisation.

10. Procédé selon la revendication 8 ou 9,
dans lequel l'ordre de déclenchement est provoqué en appuyant sur une touche prévue sur la caméra intraorale destinée à la prise de vues en continu.

11. Procédé selon la revendication 8, 9 ou 10,
dans lequel les vues prises en continu sont temporairement enregistrées sur une période de temps de 0,05 à 2 secondes, préférentiellement de 0,1 à 0,5 seconde, très préférentiellement d'environ 0,2 à environ 0,35 seconde.

12. Procédé selon une des revendications 8 à 11,
dans laquelle la position et l'orientation d'une caméra intraorale destinée à la prise des vues sont définies dans l'espace, les signaux de position et d'orientation sont transmis au dispositif de visualisation et/ou à une unité de calcul, et
les vues prises sur la base de ces signaux sont affichées à l'endroit et/ou de manière redressée.

13. Procédé selon la revendication 12,
dans lequel la position et l'orientation de la caméra sont définies par la détection du sens gravitationnel ou par la lumière.

14. Procédé selon une des revendications 8 à 11,
dans lequel des instructions de commande parlées sont recueillies afin de positionner et d'orienter la vue, sont acheminées à une unité de calcul reliée au dispositif de visualisation et
dans lequel les vues prises par la caméra sont affichées sur le dispositif de visualisation, en fonction des instructions de commande, après rotation et/ou de manière inversée.
